(19) Europäisches Patentamt / European Patent Office / Office européen des brevets

(11) **EP 3 477 648 B1**

(12) **EUROPEAN PATENT SPECIFICATION**

(45) Date of publication and mention of the grant of the patent:
**10.09.2025 Bulletin 2025/37**

(51) International Patent Classification (IPC):
**G16B 20/00** (2019.01)  **G16B 15/00** (2019.01)
**G16B 50/50** (2019.01)

(21) Application number: **18202170.9**

(22) Date of filing: **23.10.2018**

(52) Cooperative Patent Classification (CPC):
**G16B 15/00; G16B 20/00; G16B 50/50**

(54) **BIOLOGICAL SEQUENCE FINGERPRINTS**

BIOLOGISCHE SEQUENZFINGERABDRÜCKE

EMPREINTES DIGITALES DE SÉQUENCE BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2017 US 201715796679**

(43) Date of publication of application:
**01.05.2019 Bulletin 2019/18**

(73) Proprietor: **Dassault Systemes Americas Corp.**
**Waltham, MA 02451 (US)**

(72) Inventors:
• **KERMAN, Ian M.**
  **San Diego, CA 92121 (US)**
• **BRIEDIS, Kristine**
  **San Diego, CA 92121 (US)**
• **MARKEL, Scott**
  **San Diego, CA 92121 (US)**
• **ROGERS, Dave**
  **San Diego, CA 92121 (US)**

(74) Representative: **Bandpay & Greuter**
**11 rue Christophe Colomb**
**75008 Paris (FR)**

(56) References cited:
**WO-A1-01/06454**   **US-A1- 2002 072 887**
**US-A1- 2016 124 966**   **US-A1- 2017 270 240**

• **DARIUSZ PLEWCZYNSKI ET AL: "Support-vector-machine classification of linear functional motifs in proteins", JOURNAL OF MOLECULAR MODELING, SPRINGER, DE, vol. 12, no. 4, 1 March 2006 (2006-03-01), pages 453 - 461, XP019352287, ISSN: 0948-5023**
• **DAVID ROGERS ET AL: "Extended-Connectivity Fingerprints", JOURNAL OF CHEMICAL INFORMATION AND MODELING, vol. 50, no. 5, 24 May 2010 (2010-05-24), US, pages 742 - 754, XP055315446, ISSN: 1549-9596, DOI: 10.1021/ci100050t**
• **ARKAT ET AL: "General Papers ARKIVOC 2006 (ix) 211-238 ISSN 1424-6376 Page 211 Mathematical descriptors of DNA sequences: development and applications", 1 January 2006 (2006-01-01), XP055571333, Retrieved from the Internet <URL:http://www.arkat-usa.org/get-file/22721/> [retrieved on 20190319]**
• **R. JAYALAKSHMI ET AL: "Extension of molecular similarity analysis approach to classification of DNA sequences using DNA descriptors", SAR AND QSAR IN ENVIRONMENTAL RESEARCH, vol. 22, no. 1-2, 1 January 2011 (2011-01-01), GB, pages 21 - 34, XP055571340, ISSN: 1062-936X, DOI: 10.1080/1062936X.2010.528255**

## Description

BACKGROUND

Previously, the terms "DNA profiling" or "DNA fingerprinting," have been used to describe methods used in a variety of applications including criminal investigations, paternity testing, contamination detection, and testing food for accurate labeling. The fingerprinting can be done either by sequencing the DNA and using the sequence of the DNA as the fingerprint or by processing the DNA in such a way that a DNA "profile" is generated. This fingerprint is then compared to the fingerprint of a reference DNA sample. The comparison will then provide some probability that the two DNA samples are from the same source. This is an "identification" technique and typically more refers to the laboratory method rather than the comparison method.

A step beyond DNA fingerprinting is full DNA sequence comparison. Here two or more sequences are compared to each other and a similarity score is generated representing how similar the two sequences are. The most famous of these is the Basic Local Alignment Search Tool, or BLAST. There are numerous variations of BLAST designed for different applications or implementing slightly different algorithms.

Moving beyond direct sequence comparison, there are methods and databases used to identify motifs and patterns in DNA and protein sequences. Matching a particular known motif allows one to classify and, depending on the quality of the motif, assign functionality to a particular sequence. Collections of these motifs and patterns can be considered a "protein fingerprint," allowing classification of a sequence into a known class of proteins. It can also be used to identify known sequence-based structural features, such as a pocket where the protein binds to a ligand.

In the field of chemical molecular analysis, there are fingerprinting techniques in existence, but they are not applicable to biological sequences, and the existing art for biological fingerprinting is heavily dependent on comparing sequences directly or to compiled patterns of sequences (profiles). Examples of existing methods include the method for identifying an individual disclosed in the document WO 01/06454 A1 and the method for analysis of genetic material disclosed in the document US 2017/0270240 A1. These methods can be computationally expensive. BLAST, for example, runs in O(nm) time, although the modern version has many improvements that make it very efficient. These improvements involve preprocessing of the sequences and creating an index, which runs in O(n) time.

Protein fingerprints are limited to what we know about proteins; they don't allow the discovery of unknown features that may be important. This is useful for classifying and comparing proteins, but not for determining differences that may explain differences in behavior.

SUMMARY

The invention is set out in the appended set of claims.

In particular, it is provided a first computer-implemented method according to any one of claims 1 to 5, along with a corresponding computer system, computer program, and non-transitory computer-readable medium. Similarly, it is also provided a second computer-implemented method according to any one of claims 6 to 9, along with a corresponding computer system, computer program, and non-transitory computer-readable medium.

In accordance with one embodiment of the invention, features of biological sequences are represented in a fingerprint that includes a bitset, and may also include counts, strings or continuous values, for the features. The fingerprint can be used with machine learning and statistical methods. This is especially advantageous for, though not limited to, drug discovery processes. The method permits Structure-Activity Relationship (SAR) and Quantitative Structure-Activity Relationship (QSAR) studies to be performed with biological sequences.

In another embodiment in accordance with the invention, there is provided a computer system comprising: a processor; and a memory with computer code instructions stored thereon, the processor and the memory, with the computer code instructions being configured to implement a sequence evaluation module and a component feature editor module. The sequence evaluation module is configured, for each component feature of a plurality of component features to be used in a fingerprint data structure, to query a biological sequence data structure representing the biological sequence regarding a presence or value of the component feature in the biological sequence data structure. The component feature editor module is configured, for each such component feature, to add a component feature entry to the fingerprint data structure corresponding to the result of the querying of the biological sequence data structure for the component feature. At least a portion of the component feature entries of the fingerprint data structure comprise feature bits of a bitset comprising the at least a portion of the component feature entries of the fingerprint data structure.

In further, related embodiments, the sequence evaluation module may be further configured to query the biological sequence data structure to determine a value of at least one component feature entry of the fingerprint data structure that comprises a value characterizing the biological sequence as a whole. The sequence evaluation module may be further configured to query the biological sequence data structure to determine at least one component feature comprising a feature calculated or derived from the biological sequence data structure. The sequence evaluation module may be further configured to determine the feature calculated or derived from the biological sequence data structure based at least on a presence or absence of a unique sequence string appearing in a plurality of movements of a sliding window comprising neighboring units within a given distance of units of a base position unit in the biological sequence data structure. The sequence evaluation module may be further configured to determine the feature calculated or derived from the biological sequence data structure based on at least a presence or absence of a unique sequence string of a given integer length of successive units of the biological sequence data structure. The sequence evaluation module may be further configured to determine the unique sequence string by merging neighboring unique sequence strings of a smaller integer length of successive units of the biological sequence data structure to create the unique sequence string as a unique sequence of a larger integer length of successive units of the biological sequence data structure. The sequence evaluation module may be further configured to determine the feature calculated or derived from the biological sequence data structure based on at least one of: a presence or absence of at least one pattern in the biological sequence data structure, and a presence or absence of at least one pattern in at least one position of the biological sequence data structure. The sequence evaluation module may be further configured to query the biological sequence data structure to determine at least one component feature comprising a feature representing an annotation of the biological sequence. The sequence evaluation module may be further configured to query the biological sequence data structure to determine at least one component feature representing at least one of an order relationship or a distance relationship between two or more other component features of the biological sequence.

In another embodiment according to the invention, there is provided a non-transitory computer-readable medium configured to store instructions for forming a fingerprint data structure representing a biological sequence, the instructions, when loaded and executed by a processor, cause the processor to form a fingerprint data structure representing a biological sequence by: for each component feature of a plurality of component features to be used in the fingerprint data structure, querying a biological sequence data structure representing the biological sequence regarding a presence or value of the component feature in the biological sequence data structure; and adding a component feature entry to the fingerprint data structure corresponding to the result of the querying of the biological sequence data structure for the component feature. At least a portion of the component feature entries of the fingerprint data structure comprise feature bits of a bitset comprising the at least a portion of the component feature entries of the fingerprint data structure.

BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.

FIG. 1 is a schematic block diagram of a biological sequence bitset fingerprint data structure system, in accordance with an embodiment of the invention.

FIG. 2 is a schematic block diagram of a sequence evaluation module interacting with a biological sequence data structure, in accordance with an embodiment of the invention.

FIG. 3 is a schematic block diagram of a secondary feature module interacting with a biological sequence data structure, in accordance with an embodiment of the invention.

FIG. 4 is a schematic block diagram of a computer-implemented method for forming a fingerprint data structure representing a biological sequence, in accordance with an embodiment of the invention.

FIG. 5 is a schematic flow chart of method of creating a fingerprint data structure for a biological sequence, in accordance with an embodiment of the invention.

FIG. 6 is a schematic flow chart of a method of creating a bitset fingerprint data structure for a biological sequence, using bit initialization, in accordance with an embodiment of the invention.

FIG. 7 is a schematic diagram showing implementation of a sliding window technique of sequence evaluation, in accordance with an embodiment of the invention.

FIG. 8 is a schematic diagram showing implementation of a determination of unique sequence strings of different lengths, in accordance with an embodiment of the invention.

FIG. 9 is a schematic diagram showing implementation of an extended-connectivity technique of sequence evaluation, in accordance with an embodiment of the invention.

FIG. 10 is a schematic block diagram showing a biological sequence bitset fingerprint data structure interacting with a similarity evaluation module, an analysis module, a machine learning module, a searching module, and/or a metagenomics module, in accordance with an embodiment of the invention.

FIG. 11 illustrates a computer network or similar digital processing environment in which embodiments of the present invention may be implemented.

FIG. 12 is a diagram of an example internal structure of a computer (e.g., client processor/device or server computers) in the computer system of FIG. 11.

DETAILED DESCRIPTION

A description of example embodiments follows.

In accordance with one embodiment of the invention, features of biological sequences are represented in a fingerprint that includes a bitset, and may also include counts, strings or continuous values, for the features. The fingerprint can be used with machine learning and statistical methods. This is especially advantageous for, though not limited to, drug discovery processes. The method permits Structure-Activity Relationship (SAR) and Quantitative Structure-Activity Relationship (QSAR) studies to be performed with biological sequences. Because the structure of the fingerprint is not dependent on the type of sequence (for example, a DNA, RNA or protein sequence), similar machine learning and statistical methods should be able to be used regardless of the type of sequence, although the feature sets are likely not comparable between sequence types.

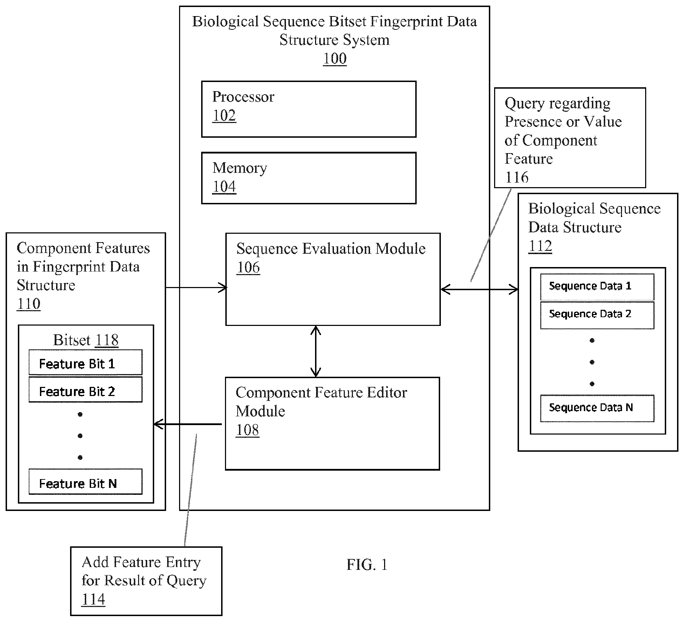

FIG. 1 is a schematic block diagram of a biological sequence bitset fingerprint data structure system 100, in accordance with an embodiment of the invention. The system 100 includes a processor 102 and a memory 104, which stores computer code instructions. The processor 102 and the memory 104, with the computer code instructions, are configured to implement a sequence evaluation module 106 and a component feature editor module 108. The sequence evaluation module 106 is configured to query 116 a biological sequence data structure 112, which represents the biological sequence, regarding a presence or value of a component feature in the biological sequence data structure 112. This is performed for each component feature that is to be used in a fingerprint data structure 110. The component feature editor module 108 is configured to add 114 a component feature entry to the fingerprint data structure 110 corresponding to the result of querying the biological sequence data structure 112 for each of the component features. At least some of the component feature entries of the fingerprint data structure 110 comprise feature bits of a bitset 118. Each bit of the bitset 118 of the fingerprint data structure 110 corresponds to a unique component feature of the biological sequence data structure 112. A value of 1 in a bit of the bitset 118 means that that feature is present in the biological sequence data structure 112, while a value of 0 means that the feature is not present in the biological sequence data structure 112.

In accordance with an embodiment of the invention, a biological sequence fingerprint data structure 110 is a collection of values representing component features of the biological sequence data structure 112. The values may indicate the presence or absence of the feature in the sequence, which can be indicated in the bitset 118. The values of the fingerprint data structure 110 can also indicate a feature's actual value, which may be a continuous number value, or a count of the number of times that a feature appears in a sequence. Whereas a bitset 118 shows whether a feature is present or not present in a biological sequence data structure 112, counts tell how many times a feature occurs in a biological sequence data structure 112, whether zero times or a number greater than zero times. In the fingerprint data structure 110, the component features may, for example, be: properties of the sequence (e.g., length); derivations of the sequence (e.g., n-mers); annotations of the sequence (e.g., single nucleotide polymorphisms or SNP's); and order and distance relationships between features (e.g., an upstream promoter region). In one example, a component feature may, for example, be the presence or absence of a pattern or motif in the biological sequence data structure, or the presence or absence of such a pattern or motif at a certain position in the biological sequence data structure. As used herein, it should be appreciated that a pattern or motif can be considered to be present, as a component feature of the biological sequence data structure, even where the pattern or motif involves ambiguities, negations or wildcards, rather than an exact match to a pattern or motif. In another example, for protein sequences, a component feature may include a feature reflecting protein/peptide crosslinking, including component features indicating the presence or absence of protein/peptide crosslinking at a given position in a protein sequence or other component features related to protein/peptide crosslinking. Component features can be represented as bits in the bitset 118 (for example, the presence or absence of such features), or as continuous values, counts, or strings, as a combination of more than one of the foregoing. In accordance with an embodiment of the invention, the fingerprint data structure 110 encapsulates the known and selected features of the sequence. Two identical sequences produce the same fingerprint, but two different sequences may or may not produce the same fingerprint depending on the features selected. Different types of fingerprint data structure 110 may be used, depending on how the component features are chosen, but the form of the fingerprint data structure 110 can include a bitset 118 regardless of which component features are chosen.

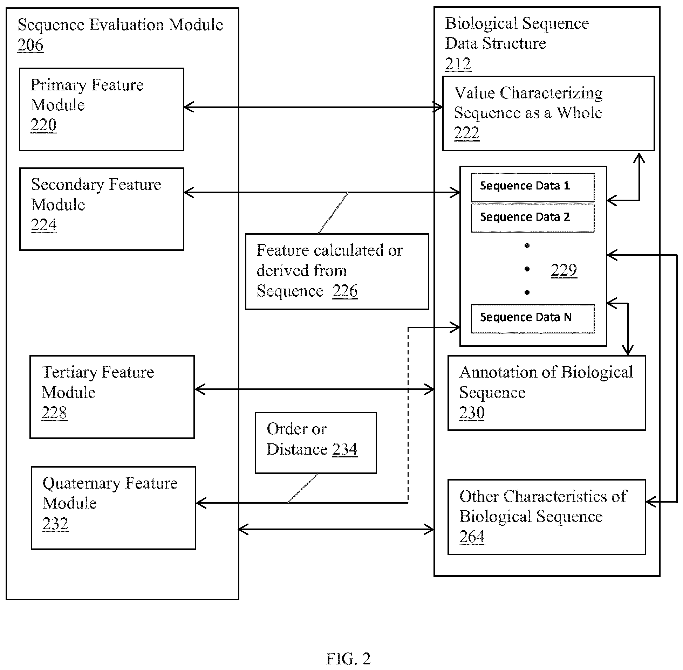

FIG. 2 is a schematic block diagram of a sequence evaluation module 206 interacting with a biological sequence data structure 212, in accordance with an embodiment of the invention. The sequence evaluation module 206 is configured to query the biological sequence data structure 212 to determine a value of at least one component feature entry of the fingerprint data structure.

The sequence evaluation module 206 of the embodiment of FIG. 2 can include a primary feature module 220 that is configured to query the biological sequence data structure 212 regarding primary features, which are features whose values 222 characterize the biological sequence as a whole. Primary features may include features such as the sequence length, the sequence's guanine-cytosine content (GC-content), codon usage bias or in the case of protein sequences, the sequence's residue content. Such values 222 characterizing the sequence as a whole 222 can be stored independently in the biological data structure 212, and, in some cases, can be themselves initially determined from sequence data 229 within the biological data structure 212 in order to characterize the biological sequence as a whole, for example, by determining the sequence's length.

The sequence evaluation module 206 of the embodiment of FIG. 2 can also include a secondary feature module 224 that is configured to query the biological sequence data structure 212 regarding secondary features, which are features calculated or derived 226 from the biological sequence data structure 212. Such features are discussed in more detail below and can, for example, include features calculated or derived from the biological sequence data structure 212 that do not merely characterize the biological sequence as a whole. For example, secondary features can include: the presence or absence of a unique sequence string appearing in a plurality of movements of a sliding window comprising neighboring units within a given distance of units of a base position unit in the biological sequence data structure; a presence or absence of a unique sequence string of a given integer length of successive units of the biological sequence data structure; a unique sequence string created by merging neighboring unique sequence strings of a smaller integer length of successive units of the biological sequence data structure to create a unique sequence of a larger integer length of successive units; a presence or absence of at least one pattern in at least one position of the biological sequence data structure; and a presence or absence of at least one sequence string in the biological sequence data structure.

The sequence evaluation module 206 of the embodiment of FIG. 2 can also include a tertiary feature module 228 that is configured to query the biological sequence data structure 212 regarding tertiary features, which are features representing an annotation of the biological sequence 230. Such tertiary features can, for example, include: annotations that identify single nucleotide polymorphisms (SNP's) in a sequence; annotations that identify the presence of sequence patterns indicating some functionality, such as transcription factor binding; or results from querying the sequence against a protein fingerprint library, for example, Pfam or InterPro (both databases of the European Molecular Biology Laboratory-European Bioinformatics Institute of Hinxton, Cambridgeshire, United Kingdom). In these cases, the fingerprint data structure 110 (see FIG. 1) can, for example, indicate whether the biological sequence data structure 212 has the feature or does not have the feature. Such annotations 230 can be stored independently in the biological data structure 212, and, in some cases, can be themselves initially determined from sequence data 229 within the biological data structure 212, for example by initially querying the sequence against a protein fingerprint library.

The sequence evaluation module 206 of the embodiment of FIG. 2 can also include a quaternary feature module 232 that is configured to query the biological sequence data structure 212 regarding quaternary features, which are features representing at least one of an order relationship or a distance relationship 234 between two or more other component features of the biological sequence. An example of this would be specifying that one gene feature is located 54 base pairs (bp) away from another gene feature. Another example could be that gene B is located between gene A and gene C or that gene Z follows gene Y in the sequence, but with no distances between them specified. When distances are specified, ranges can also be allowed. Such quaternary features can be stored in a bitset 118 (the presence or absence of such an order or distance relationship 234) or as a count, continuous value or string.

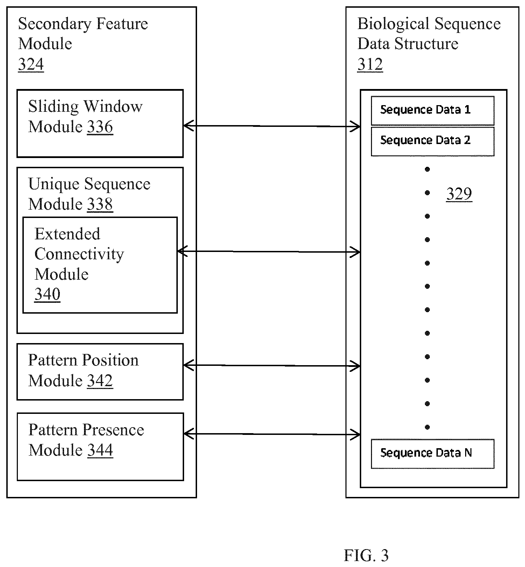

FIG. 3 is a schematic block diagram of a secondary feature module 324 interacting with a biological sequence data structure 312, in accordance with an embodiment of the invention.

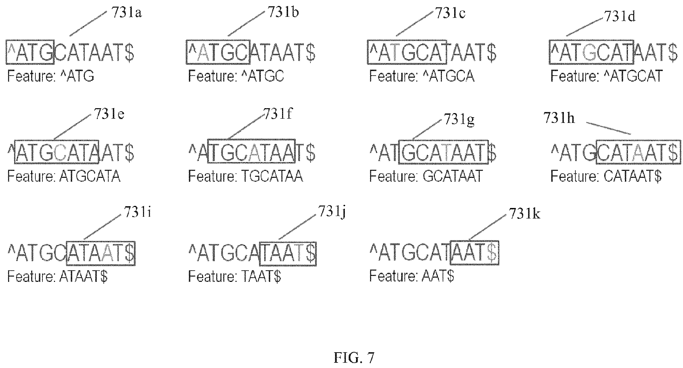

The secondary feature module 324 of the embodiment of FIG. 3 can, for example, include a sliding window module 336 that is configured to determine a feature calculated or derived from the biological sequence data structure 312 based at least on a presence or absence of a unique sequence string appearing in a plurality of movements of a sliding window comprising neighboring units within a given distance of units of a base position unit in the biological sequence data structure 312. The sliding window module 336 can perform this using sequence data 329, and is illustrated further, below, in connection with FIG. 7.

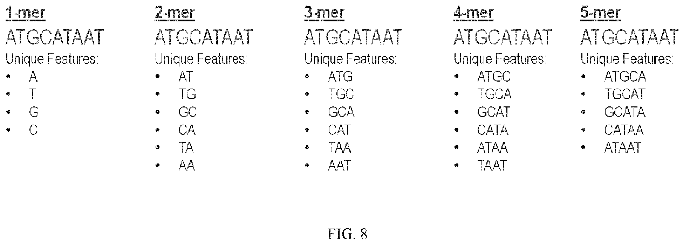

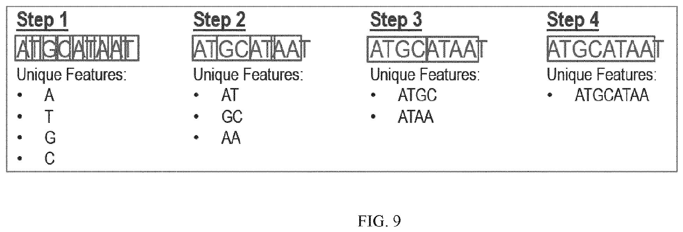

The secondary feature module 324 of the embodiment of FIG. 3 can, for example, also include a unique sequence module 338, which is configured to determine the feature calculated or derived from the biological sequence data structure 312 based on at least a presence or absence of a unique sequence string of a given integer length of successive units of the biological sequence data structure 312. The unique sequence module 338 can perform this using sequence data 329, and is illustrated further, below, in connection with FIG. 8. In a further example, the unique sequence string can be determined by an extended connectivity module 340, by merging neighboring unique sequence strings of a smaller integer length of successive units of the biological sequence data structure 312 to create the unique sequence string as a unique sequence of a larger integer length of successive units of the biological sequence data structure 312. The extended connectivity module 340 can perform this using sequence data 329, and is illustrated further, below, in connection with FIG. 9.

The secondary feature module 324 of the embodiment of FIG. 3 can, for example, also include a pattern position module 342, which is configured to determine a feature calculated or derived from the biological sequence data structure 312 based on a presence or absence of at least one pattern in at least one position of the biological sequence data structure 312. The secondary feature module 324 can perform this using sequence 329. For example, the secondary feature module can determine:

1. Whether Residue/Base X is at Position N in biological sequence data structure 312.

2. Whether Residue/Base X is NOT at Position N in biological sequence data structure 312.

3. Whether Residues/Bases X,Y and Z, or X, Y or Z, are at Position N in biological sequence data structure 312.

4. Whether Residues/Bases X,Y and Z (or X, Y or Z) are NOT at Position N in biological sequence data structure 312.

In addition, the secondary feature module 324 of the embodiment of FIG. 3 can, for example, also include a pattern presence module 344, which is configured to determine a feature calculated or derived from the biological sequence data structure 312 based on a presence or absence of at least one pattern, such as at least one sequence string, in the biological sequence data structure 312. Here, a component feature of the fingerprint data structure 110 (see FIG. 1) is a pattern, and a bit of the bitset 118 (see FIG. 1) can be set based on whether the feature matches the pattern or not. Such a feature could be a match to a Regular Expression pattern. Here, it should be appreciated that a match to a pattern or motif can be considered to be present, as a component feature of the biological sequence data structure, even where the pattern or motif involves ambiguities, negations or wildcards, rather than an exact match to a pattern or motif. Metadata (or qualifiers) in fingerprint data structure (see 110 in FIG. 1) for a component feature can be set to include the pattern, or a pattern identifier. In one example, the pattern presence module 344 can determine, using sequence data 329:

1. Whether Sequence String XYZ is in biological sequence data structure 312;

2. Whether Sequence String XYZ is NOT in biological sequence data structure 312.

Ambiguities, negations or wildcards, rather than an exact match to a pattern or motif, can also be used by the pattern presence module 344 and pattern position module 342. More generally, Regular Expression pattern matching can be performed in accordance with an embodiment of the invention, including the use of ambiguities, negations or wildcards. For example, Regular Expression pattern matching can be used with the syntax of any of the IEEE Portable Operating System Interface (POSIX) family of standards, including any of the syntax of Basic Regular Expressions (BRE), Extended Regular Expressions (ERE) or Simple Regular Expressions (SRE), such as those based on IEEE Std 1003.1-2008, 2016 Edition. Some examples of Regular Expression pattern matching that can be used to match patterns in a biological sequence data structure 312 are as follows, without limitation, where it will be appreciated that reference to a "character" or "letter" is here used to refer to an element, such as an element for a base or residue, in sequence data 329 of a biological sequence data structure 312:

.at matches any three-character string ending with "at", including "hat", "cat", and "bat".
[hc]at matches "hat" and "cat".
[a-z] specifies a range which matches any letter from "a" to "z". These forms can be mixed: [abcx-z] matches "a", "b", "c", "x", "y", or "z", as does [a-cx-z]
[^b] at matches all strings matched by .at except "bat".
[^hc]at matches all strings matched by .at other than "hat" and "cat".
^[hc]at matches "hat" and "cat", but only at the beginning of the string.
[hc]at$ matches "hat" and "cat", but only at the end of the string.
s.* matches s followed by zero or more characters, for example: "s" and "saw" and "seed".
a{3,5} matches only "aaa", "aaaa", and "aaaaa".

In addition, in the embodiment of FIG. 3, it will be appreciated that logical permutations of examples (1) through (4), given above for the pattern position module 342, and of examples (1) and (2), given above for the pattern presence module 344, can be used, such as by using both pattern position module 342 and pattern presence module 344, or a single module that includes both functionalities. Logical combinations of more than one inquiry can be performed using Boolean logical expressions, such as AND, OR and NOT. For example, the secondary feature module 324 can determine features such as:

1. Whether Residues/Bases X are at Position N AND Residues/Bases Y are at Position M in biological sequence data structure 312.

2. Whether Residue/Base X is NOT at Position N in biological sequence data structure 312 AND Whether Residue/Base Y is NOT at Position M in biological sequence data structure 312.

3. Whether Residues/Bases X,Y and Z, or X, Y or Z, are at Position N in biological sequence data structure 312 AND Whether Residues/Bases X,Y and Z, or X, Y or Z, are at Position M in biological sequence data structure 312.

4. Whether Residues/Bases X,Y and Z (or X, Y or Z) are NOT at Position N in biological sequence data structure 312 AND Whether Residues/Bases X,Y and Z (or X, Y or Z) are NOT at Position M in biological sequence data structure 312.

5. Whether Sequence String XYZ is in biological sequence data structure 312 AND Whether Sequence String ABC is in biological sequence data structure 312.

> 6. Whether Sequence String XYZ is NOT in biological sequence data structure 312 AND Whether Sequence String ABC is NOT in biological sequence data structure 312.

It will be appreciated that other permutations and combinations of such inquiries may be performed using secondary feature module 324. In addition, in an embodiment according to the invention, such as in the secondary feature module 324, pattern position module 342 and/or pattern presence module 344, one or more pattern matching techniques may be used in accordance with the teachings of Markel S., Rajapakse V., Pattern Matching, in In Silico Technology in Drug Target Identification and Validation Leon D, Markel S (Editors), Marcel Dekker, 2006.

In addition, it should be appreciated that, in accordance with an embodiment of the invention, component features may be included in a fingerprint data structure 110 (see FIG. 1) that fit into none of the above categories of primary, secondary, tertiary and quaternary features, or that fit, to some extent, in more than one of those categories, and may be evaluated by using the sequence evaluation module 106 to query the biological sequence data structure 112 regarding the presence or value of such component features. A feature bit in a bitset, a count, a string or a continuous value may be included corresponding to such component features. Such features can, for example, be included in an additional field 264 (see FIG. 2) of biological sequence data structure 212 for other characteristics of biological sequences and evaluated by sequence evaluation module 206, and/or can themselves be derived from sequence data 229.

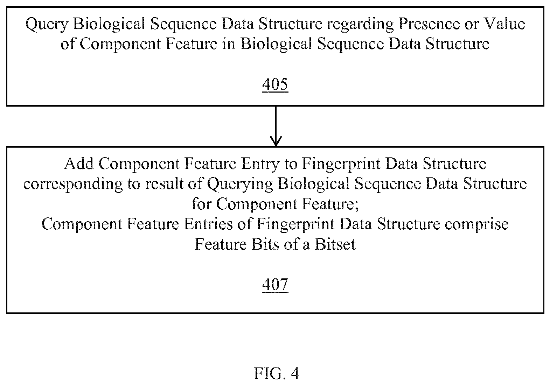

FIG. 4 is a schematic block diagram of a computer-implemented method for forming a fingerprint data structure representing a biological sequence, in accordance with an embodiment of the invention. The computer-implemented method comprises, 405, for each component feature of a plurality of component features to be used in the fingerprint data structure, querying a biological sequence data structure representing the biological sequence regarding a presence or value of the component feature in the biological sequence data structure. A component feature entry is added, 407, to the fingerprint data structure, corresponding to the result of the querying of the biological sequence data structure for the component feature. At least a portion of the component feature entries of the fingerprint data structure comprise feature bits of a bitset comprising the at least a portion of the component feature entries of the fingerprint data structure.

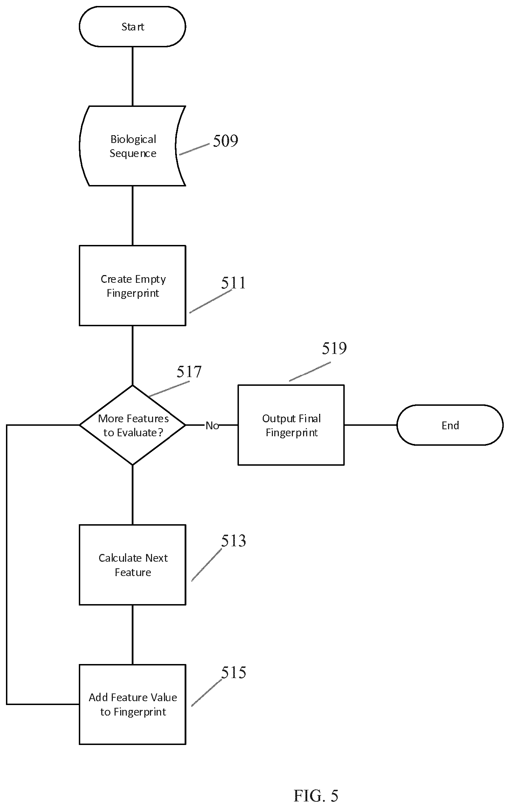

FIG. 5 is a schematic flow chart of method of creating a fingerprint data structure for a biological sequence, in accordance with an embodiment of the invention. Given a set of features, an empty fingerprint is created 511. The biological sequence 509 is queried 513 as to whether or not it contains that feature or in some cases, what that value of that feature may be. The result of this operation is then added 515 to the fingerprint and the next feature is evaluated 513. To add 515 the feature to the feature, where the feature is a feature to be recorded in a bitset, a bit of the bitset is set regarding whether the feature is present or not; whereas, for other features, a count, continuous value or string is added to the fingerprint for that feature. If there are no more features to evaluate 517, the final fingerprint is output 519.

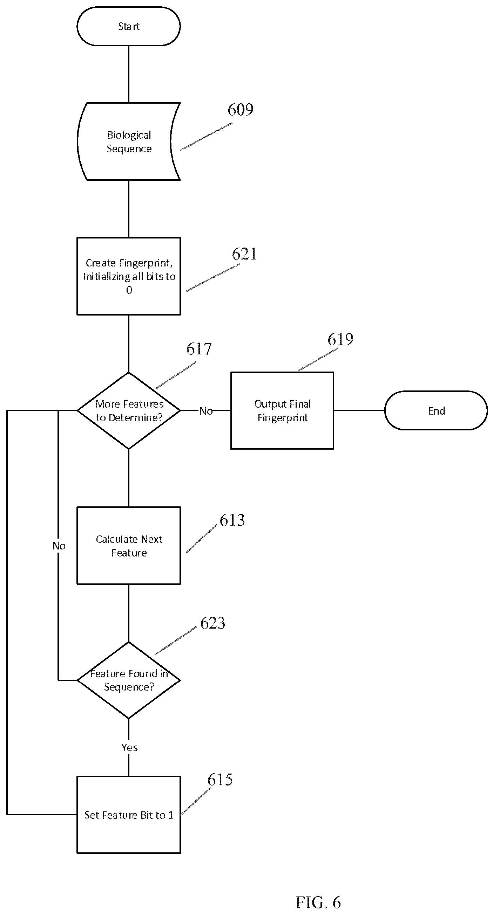

FIG. 6 is a schematic flow chart of a method of creating a bitset fingerprint data structure for a biological sequence, using bit initialization, in accordance with an embodiment of the invention. Here, in one embodiment, the fingerprint is initially created 621 by initializing all bits of the bitset to zero (0), indicating the absence of a feature. The biological sequence 609 is queried 613 as to whether or not it contains that feature, and if the feature is found in the sequence 623, the feature bit is set 615 to one (1). The next feature is evaluated 613. If there are no more features to evaluate 617, the final fingerprint is output 619.

FIG. 7 is a schematic diagram showing implementation of a sliding window technique of sequence evaluation, in accordance with an embodiment of the invention. In this embodiment a fingerprint is created based on each sequence position's neighbors within a given plus or minus distance window within the biological sequence data structure 312 (of FIG. 3). This can, for example, be performed using sliding window module 336 (of FIG. 3). For example, with reference to sliding window 731f, it can be seen that sequence position A in the center of the sliding window, surrounded by neighbors within plus or minus three sequence positions, namely the three neighbors T, G and C to the left of position A and the three neighbors T, A and A to the right of position A. The sliding window travels across the sequence from left to right, beginning in position 731a, and continuing through positions 731b through 731k. Features are defined as the unique sequence appearing in each movement of the sliding window. It will be noticed, however, that as the sliding window enters the sequence from the left (in FIG. 7, starting with 731a), and as it leaves the sequence to the right (in FIG. 7, ending with 731k), the number of items in the sliding window is reduced. Thus, position 731a contains only three positions, position 731b contains four, position 731c contains five, position 731d contains six, and position 731e contains seven. The seven positions continue as the sliding window slights to the right in positions 731f through 731h, but beginning with position 731i the sliding window contains six, five, four etc. positions as the sliding window slides off the sequence to the right. It can be seen that, in this example, the first and last positions appear in four features (731a-731d and 731h-731k), whereas the middle positions appear in seven (731c through 731i). Therefore, a variation of the sliding window technique, in one embodiment, is to use, for example, three "anchor" characters, rather than just one anchor character, at the beginning and/or ending of the sequence. "^" and "$" are the anchor characters indicating the beginning and end, respectively, of the sequence in FIG. 7. Thus, a sequence could be recorded in the data structure as: ^^^ATGCATAAT$$$ instead of ^ATGCATAAT$. This would allow equal capturing of beginning and ending bases/residues compared to other bases/residues that are in middle positions (such as positions 731e through 731h in FIG. 7). In addition, a wildcard symbol can be used in accordance with the embodiment of FIG. 7 and other embodiments of the invention taught herein, in order to symbolize that any residue or base, or any plurality of residues or bases, can be present at the location of the wildcard symbol and still be considered to match a pattern.

FIG. 8 is a schematic diagram showing implementation of a determination of unique sequence strings of different lengths, in accordance with an embodiment of the invention. Here, for example, the unique sequence module 338 of FIG. 3 can be used to go through the biological sequence data structure 312 of FIG. 3 and determine all of the unique N-mers in a sequence for a given N or range of N, such as the 1-mer, 2-mer, 3-mer, 4-mer and 5-mer shown in FIG. 8. In the 1-mer in FIG. 8, the unique features are A, T, G and C; whereas in the 2-mer, the unique features are AT, TG, GC, CA, TA and AA; in the 3-mer, the unique features are ATG, TGC, GCA, CAT, TAA and AAT; and so forth. Once all of the unique n-mers in a sequence are found, each n-mer is used as a component feature of the fingerprint data structure, and its presence or absence can, for example, be used as a bit in a bitset (such as 118 of FIG. 1). It is possible that, for low complexity sequences, or very long sequences, this technique may be improved by using feature counts, instead of (or in addition to) setting bits in a bitset, due to feature collisions in such sequences.

FIG. 9 is a schematic diagram showing implementation of an extended-connectivity technique of sequence evaluation, in accordance with an embodiment of the invention. This technique can, for example, be implemented using extended connectivity module 340 of FIG. 3, based on biological sequence data structure 312. This technique involves merging neighboring unique sequence strings of a smaller integer length of successive units of the biological sequence data structure 312 to create the unique sequence string as a unique sequence of a larger integer length of successive units of the biological sequence data structure 312. As an example, with reference to FIG. 9, the technique starts with a set of n-mers and then progressively joins them into larger n-mers. First, beginning with individual bases/residues, the unique sequences; here, in Step 1 of FIG. 9, the unique features of the individual bases/residues are A, T, G and C. Next, two adjacent sequences of the same size are merged into each other, as in step 2, and each unique sequence created is a feature. For example, in step 2, the new unique features are AT, GC and AA. This process continues for progressively higher sizes of n-mers, continuing, for example, with n-mers of length four and eight in steps 3 and 4 of FIG. 9. The unique strings so determined are used as component features of the fingerprint data structure, for example by setting a bit in a bitset depending on the presence or absence of such as unique string, or by using a count, string or continuous value for the unique sequences so created. In one example, any bases/residues/merged groups not merged are dropped, and merging is started with the first position. However, other variations on this technique can include alternatives to merging with the first position, such as starting merging at the last position; starting merging at both the first and last position, and meeting in the middle; or repeating merging twice, once from the first position, once from the last position. Also, the handling of unmerged bases/residues/groups can be changed, for example by merging the unmerged bases/residues/groups into the most adjacent group. In accordance with an embodiment of the invention, such a technique of extended-connectivity sequence evaluation can use any of the features taught in David Rogers and Mathew Hahn, Extended-Connectivity Fingerprints, Journal of Chemical Information and Modeling 2010 50 (5), 742-754. DOI: 10.1021/ci100050t. http://pubs.acs.org/doi/abs/10.1021/ci100050t.

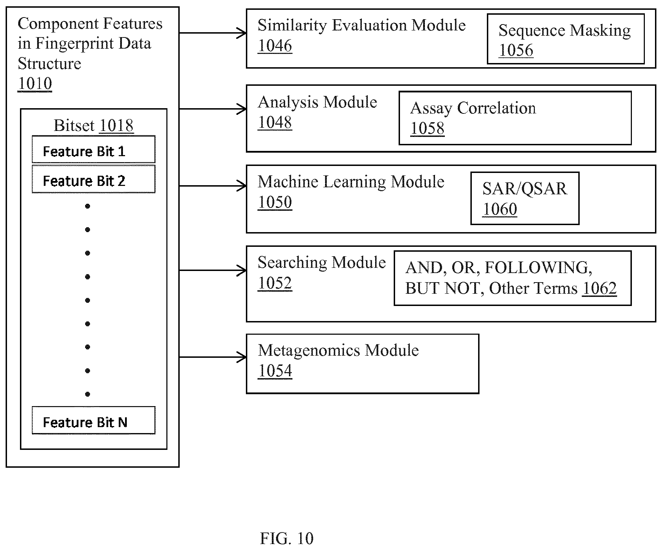

FIG. 10 is a schematic block diagram showing a biological sequence bitset 1018 fingerprint data structure 1010 interacting with a similarity evaluation module 1046, an analysis module 1048, a machine learning module 1050, a searching module 1052, and/or a metagenomics module 1054, in accordance with an embodiment of the invention. An embodiment according to the invention can, for example, include one or more of such modules, in addition to components shown elsewhere.

In the embodiment of FIG. 10, a similarity evaluation module 1046 can be used to determine how similar a sequence is to other sequences in a database. Features in the fingerprint data structure 1010 can be hashed into a unique value representing a bit in the bitset 1018, and the fingerprint 1010 can be "yes/no" for presence of features, or the fingerprint data structure 1010 can include a count of features, a continuous value or a string. The similarity evaluation module 1046 can include a sequence masking module 1056 that allows masking of sequences so that only sequences of interest are represented in the fingerprint; for example, one could mask an antibody sequence so that only the CDR3 region of an antibody sequence is captured. In accordance with an embodiment of the invention, the fingerprints of two different biological sequence data structures can, for example, be compared by comparing the value of each bit in the bitset 1018 for each fingerprint. This can, for example, be performed by taking the Tanimoto distance between the two fingerprints to determine the similarity between the two. Here, the Tanimoto distance is defined based on a technique given in David J. Rogers and Taffee T. Tanimoto (1960), "A Computer Program for Classifying Plants," Science 132 (3434): 1115-1118. In particular, the Tanimoto distance can be determined as:

$$T_s(X,Y) = \frac{\sum_i (X_i \wedge Y_i)}{\sum_i (X_i \vee Y_i)}$$

where the similarity ratio $T_s$ is given over bitmaps, where each bit of a fixed-size array represents the presence or absence of a characteristic being modelled, with samples X and Y being bitmaps, $X_i$ being the i-th bit of X, and $\wedge$ and $\vee$ are the bitwise "and" and "or" operators respectively. Here, the concept of bitmaps is instead used with bits in a bitset of a fingerprint data structure in accordance with an embodiment of the present invention. If each sample is modelled instead as a set of attributes, this value is equal to the Jaccard coefficient of the two sets, as defined below.

It will be appreciated that other techniques suitable to determine similarity or distance between bitsets or other feature components of fingerprint data structures can be used, including techniques that compare similarity or distance between counts, strings and continuous values. For example, the Jaccard Similarity Coefficient (or it complement) may be used, which is defined as the size of the intersection divided by the size of the union of the sample sets, or:

$$J(A, B) = \frac{|A \cap B|}{|A \cup B|} = \frac{|A \cap B|}{|A| + |B| - |A \cap B|}.$$

for sets A, B, where, if both A and B are empty, we define J(A,B) = 1, and:

$$0 \leq J(A, B) \leq 1.$$

In the embodiment of FIG. 10, an analysis module 1048 can be used to perform analysis on the fingerprint data structure 1010. For example, an assay correlation module 1058 can be used to determine what sequence bits or other feature components of the fingerprint data structure 1010 are correlated with assay results.

In addition, in the embodiment of FIG. 10, a machine learning module 1050 can be used to determine what sequence bits or other component features of the fingerprint data structure 1010 are important in the sequence. For example, a Structure-Activity Relationship (SAR) or Quantitative Structure-Activity Relationship (QSAR) module 1060 can be used to analyze the fingerprint data structure 1010 to determine what component features of the fingerprint data structure 1010 are important in the biological sequence data structure. The machine learning module 1050 can also perform Bayesian learning and other techniques on the fingerprint data structure 1010.

Further, in the embodiment of FIG. 10, a searching module 1052 can be used to perform searching on fingerprint data structure 1010. For example, a search logic module 1062 can be used to search the fingerprint data structure 1010 using terms such as AND, OR, FOLLOWING, BUT NOT, and other search terms. Inquiries such as the following may be performed: What sequences have [bit A] and [bit B] in the sequence? What sequences have [bit B] following [bit A] in the sequence? What sequences have [bit A], but not [bit B] in the sequence? It will be appreciated that other searches can be performed.

In addition, in the embodiment of FIG. 10, a metagenomics module 1054 can be used to perform a metagenomics analysis on fingerprint data structure 1010. Such a module 1054 can, for example, determine which component features of the fingerprint data structure 1010, such as which bits of the bitset 1018, are represented in the biological sequence data structures.

In accordance with an embodiment of the invention, after performing one or more of a similarity evaluation using module 1046, an analysis using module 1048, a machine learning using module 1050, a search using module 1052 or a metagenomics analysis using module 1054, an embodiment according to the invention includes selecting one or more biological sequences based on the results of such analysis to use as the basis for synthesis or discovery of a drug, for improving the results of an assay, and to perform one or more alterations or additions to a production process utilizing a biological sequence, and other biological process improvements or alterations, consistent with teachings herein.

As used herein, a "bitset" corresponding to a biological sequence data structure includes feature bits in which each bit corresponds to a unique component feature of the biological sequence data structure, and in which one value of a bit means that the feature is present in the biological sequence data structure, and another value of the bit means that the feature is not present in the biological sequence data structure.

Although embodiments have been described herein in which a fingerprint data structure 1010 (see FIG. 1, for example) can include a bitset 1018 in addition to one or more other feature components, such as counts, strings and continuous values, it should be appreciated that, in some embodiments, the fingerprint data structure 1010 can include only a bitset 1018 of component features.

As used here, a "biological sequence" is a sequence including a nucleic acid or a protein. As used herein, "nucleic acid" refers to a macromolecule composed of chains (a polymer or an oligomer) of monomeric nucleotide. The most common nucleic acids are deoxyribonucleic acid (DNA) and ribonucleic acid (RNA). It should be further understood that the present invention can be used for biological sequences containing artificial nucleic acids such as peptide nucleic acid (PNA), morpholino, locked nucleic acid (LNA), glycol nucleic acid (GNA) and threose nucleic acid (TNA), among others. In various embodiments of the present invention, nucleic acids can be derived from a variety of sources such as bacteria, virus, humans, and animals, as well as sources such as plants and fungi, among others. The source can be a pathogen. Alternatively, the source can be a synthetic organism. Nucleic acids can be genomic, extrachromosomal or synthetic. Where the term "DNA" is used herein, one of ordinary skill in the art will appreciate that the methods and devices described herein can be applied to other nucleic acids, for example, RNA or those mentioned above. In addition, the terms "nucleic acid," "polynucleotide," and "oligonucleotide" are used herein to include a polymeric form of nucleotides of any length, including, but not limited to, ribonucleotides or deoxyribonucleotides. There is no intended distinction in length between these terms. Further, these terms refer only to the primary structure of the molecule. Thus, in certain embodiments these terms can include triple-, double- and single-stranded DNA, PNA, as well as triple-, double- and single-stranded RNA. They also include modifications, such as by methylation and/or by capping, and unmodified forms of the polynucleotide. More particularly, the terms "nucleic acid," "polynucleotide," and "oligonucleotide," include polydeoxyribonucleotides (containing 2-deoxy-D-ribose), polyribonucleotides (containing D-ribose), any other type of polynucleotide which is an N- or C-glycoside of a purine or pyrimidine base, and other polymers containing nonnucleotidic backbones, for example, polyamide (e.g., peptide nucleic acids (PNAs)) and polymorpholino (commercially available from Anti-Virals, Inc., Corvallis, Oreg., U.S.A., as Neugene) polymers, and other synthetic sequence-specific nucleic acid polymers providing that the polymers contain nucleobases in a configuration which allows for base pairing and base stacking, such as is found in DNA and RNA.

As used herein, a "protein" is a biological molecule consisting of one or more chains of amino acids. Proteins differ from one another primarily in their sequence of amino acids, which is dictated by the nucleotide sequence of the encoding gene. A peptide is a single linear polymer chain of two or more amino acids bonded together by peptide bonds between the carboxyl and amino groups of adjacent amino acid residues; multiple peptides in a chain can be referred to as a polypeptide. Proteins can be made of one or more polypeptides. Shortly after or even during synthesis, the residues in a protein are often chemically modified by posttranslational modification, which alters the physical and chemical properties, folding, stability, activity, and ultimately, the function of the proteins. Sometimes proteins have non-peptide groups attached, which can be called prosthetic groups or cofactors.

It will be appreciated, in addition, that a biological sequence can include non-natural bases and residues, for example, non-natural amino acids inserted into a biological sequence.

In an embodiment according to the invention, processes described as being implemented by one processor may be implemented by component processors, and/or a cluster of processors, configured to perform the described processes, which may be performed in parallel synchronously or asynchronously. Such component processors may be implemented on a single machine, on multiple different machines, in a distributed fashion in a network, or as program module components implemented on any of the foregoing.

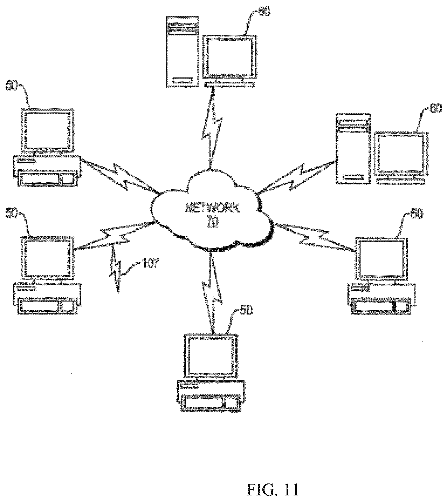

FIG. 11 illustrates a computer network or similar digital processing environment in which embodiments of the present invention may be implemented. Client computer(s)/devices 50 and server computer(s) 60 provide processing, storage, and input/output devices executing application programs and the like. The client computer(s)/devices 50 can also be linked through communications network 70 to other computing devices, including other client devices/processes 50 and server computer(s) 60. The communications network 70 can be part of a remote access network, a global network (e.g., the Internet), a worldwide collection of computers, local area or wide area networks, and gateways that currently use respective protocols (TCP/IP, Bluetooth®, etc.) to communicate with one another. Other electronic device/computer network architectures are suitable.

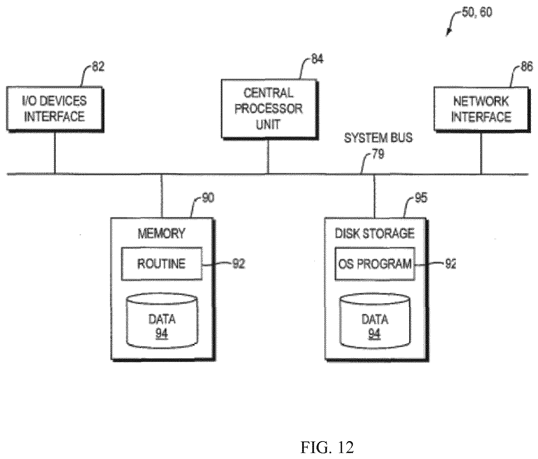

FIG. 12 is a diagram of an example internal structure of a computer (e.g., client processor/device 50 or server computers 60) in the computer system of FIG. 11. Each computer 50, 60 contains a system bus 79, where a bus is a set of hardware lines used for data transfer among the components of a computer or processing system. The system bus 79 is essentially a shared conduit that connects different elements of a computer system (e.g., processor, disk storage, memory, input/output ports, network ports, etc.) that enables the transfer of information between the elements. Attached to the system bus 79 is an I/O device interface 82 for connecting various input and output devices (e.g., keyboard, mouse, displays, printers, speakers, etc.) to the computer 50, 60. A network interface 86 allows the computer to connect to various other devices attached to a network (e.g., network 70 of FIG. 11). Memory 90 provides volatile storage for computer software instructions 92 and data 94 used to implement an embodiment of the present invention (e.g., sequence evaluation module 106, component feature editor module 108, primary feature module 220, secondary feature module 224, tertiary feature module 228, quaternary feature module 232, sliding window module 336, unique sequence module 338, extended connectivity module 340, pattern position module 342, pattern presence module 344, similarity evaluation module 1046, analysis module 1048, machine learning module 1050, searching module 1052 and metagenomics module 1054, detailed herein). Disk storage 95 provides non-volatile storage for computer software instructions 92 and data 94 used to implement an embodiment of the present invention. A central processor unit 84 is also attached to the system bus 79 and provides for the execution of computer instructions.

In one embodiment, the processor routines 92 and data 94 are a computer program product (generally referenced 92), including a non-transitory computer-readable medium (e.g., a removable storage medium such as one or more DVD-ROM's, CD-ROM's, diskettes, tapes, etc.) that provides at least a portion of the software instructions for the invention system. The computer program product 92 can be installed by any suitable software installation procedure, as is well known in the art. In another embodiment, at least a portion of the software instructions may also be downloaded over a cable communication and/or wireless connection. In other embodiments, the invention programs are a computer program propagated signal product embodied on a propagated signal on a propagation medium (e.g., a radio wave, an infrared wave, a laser wave, a sound wave, or an electrical wave propagated over a global network such as the Internet, or other network(s)). Such carrier medium or signals may be employed to provide at least a portion of the software instructions for the present invention routines/program 92.

In alternative embodiments, the propagated signal is an analog carrier wave or digital signal carried on the propagated medium. For example, the propagated signal may be a digitized signal propagated over a global network (e.g., the Internet), a telecommunications network, or other network. In one embodiment, the propagated signal is a signal that is transmitted over the propagation medium over a period of time, such as the instructions for a software application sent in packets over a network over a period of milliseconds, seconds, minutes, or longer.

While example embodiments have been particularly shown and described, it will be understood by those skilled in the art that various changes in form and details may be made therein without departing from the scope of the embodiments encompassed by the appended claims.

**Claims**

1.  A computer-implemented method for forming a fingerprint data structure representing a biological sequence, the computer-implemented method comprising:

    for each component feature of a plurality of component features to be used in the fingerprint data structure, querying a biological sequence data structure representing the biological sequence to determine a value of the component feature in the biological sequence data structure, the value indicating:

      - a presence or absence of the component feature in the biological sequence, or
      - an actual value of the component feature; and

    adding a component feature entry to the fingerprint data structure corresponding to the result of the querying of the biological sequence data structure for the component feature, the component feature entry having the determined value of the component feature;
    at least a portion of the component feature entries added in the fingerprint data structure being indicated in feature bits of a bitset, each feature bit corresponding to a respective component feature having a value indicating a presence or absence of the respective component feature in the biological sequence,
    wherein the component features include at least one of: properties of the biological sequence, derivations of the biological sequence, annotations of the biological sequence and order and distance relationships between features,
    wherein at least one component feature is:

      (i) a unique sequence string appearing in a plurality of movements of a sliding window comprising neighboring units within a given distance of units of a base position unit in the biological sequence data structure; or
      (ii) a unique sequence string of a given integer length of successive units of the biological sequence data structure, wherein optionally the unique sequence string comprises a unique sequence string of a larger given integer length of successive units of the biological sequence data structure created by merging neighboring unique sequence strings of a smaller integer length of successive units of the biological sequence data structure; or
      (iii) at least one of: at least one pattern in the biological sequence data structure, and at least one pattern in at least one position of the biological sequence data structure.

2.  The computer-implemented method of Claim 1, wherein a value of at least one component feature entry of the fingerprint data structure comprises at least one of: a count of the feature in the biological sequence data structure; a string representing the at least one component feature entry; and a continuous number value representing the at least one component feature entry.

3. The computer-implemented method of Claim 1 or 2, wherein a value of at least one component feature entry of the fingerprint data structure comprises a value characterizing the biological sequence as a whole.

4. The computer-implemented method of any one of Claims 1 to 3, wherein at least one component feature of the fingerprint data structure comprises a feature representing an annotation of the biological sequence.

5. The computer-implemented method of any one of Claims 1 to 4, wherein at least one component feature of the fingerprint data structure comprises a feature representing at least one of an order relationship or a distance relationship between two or more other component features of the biological sequence.

6. A computer implemented method for comparing a first biological sequence with a second biological sequence, the method comprising:

    forming a first fingerprint data structure representing the first biological sequence, and a second fingerprint data structure representing the second biological sequence with the computer implemented method of any one of Claims 1 to 5; and
    comparing the first fingerprint data structure and the second fingerprint data structure by determining similarity or distance between the feature components of the first fingerprint data structure and the second fingerprint data structure.

7. The computer implemented method of claim 6, wherein the determining of similarity or distance is based on the Jaccard Similarity Coefficient of the first fingerprint data structure and the second fingerprint data structure.

8. The computer implemented method of claim 7, wherein the determining of similarity or distance comprises determining a distance by comparing the value of each bit in a first bitset of the first biological sequence with the value of each bit in a second bitset of the second biological sequence, the distance representing similarity between the first bitset and the second bitset.

9. The computer implemented method of claim 8, wherein the distance is a Tanimoto distance.

10. A computer system comprising:

    a processor; and
    a memory with computer code instructions stored thereon, the processor and the memory, with the computer code instructions being configured to implement the method of any one of Claims 1 to 5.

11. A computer system comprising:

    a processor; and
    a memory with computer code instructions stored thereon, the processor and the memory, with the computer code instructions being configured to implement the method of any one of Claims 6 to 9.

12. A computer program comprising instructions for causing a processor to perform the method of any one of Claims 1 to 5.

13. A computer program comprising instructions for causing a processor to perform the method of any one of Claims 6 to 9.

14. A non-transitory computer-readable medium having recorded thereon the computer program of Claim 12.

15. A non-transitory computer-readable medium having recorded thereon the computer program of Claim 13.

**Patentansprüche**

1. Computerimplementiertes Verfahren zum Bilden einer Fingerabdruckdatenstruktur, die eine biologische Sequenz darstellt, wobei das computerimplementierte Verfahren umfasst:

für jedes Komponentenmerkmal einer Mehrzahl von Komponentenmerkmalen, die in der Fingerabdruckdatenstruktur zu verwenden sind, Abfragen einer Datenstruktur biologischer Sequenz, die die biologische Sequenz darstellt, um einen Wert des Komponentenmerkmals in der Datenstruktur biologischer Sequenz zu ermitteln, wobei der Wert angibt:

- ein Vorhandensein oder Nichtvorhandensein des Komponentenmerkmals in der biologischen Sequenz, oder
- einen tatsächlichen Wert des Komponentenmerkmals; und

Hinzufügen eines Komponentenmerkmalseintrags zur Fingerabdruckdatenstruktur entsprechend dem Ergebnis des Abfragens der Datenstruktur biologischer Sequenz für das Komponentenmerkmal, wobei der Komponentenmerkmalseintrag den ermittelten Wert des Komponentenmerkmals aufweist;

wobei mindestens ein Teil der in die Fingerabdruckdatenstruktur hinzugefügten Komponentenmerkmaleinträge in Merkmalsbits eines Bitsatzes angegeben ist, wobei jedes Merkmalsbit, das einem jeweiligen Komponentenmerkmal entspricht, einen Wert aufweist, der ein Vorhandensein oder Nichtvorhandensein des jeweiligen Komponentenmerkmals in der biologischen Sequenz angibt;

wobei die Komponentenmerkmale mindestens eines aufweisen aus: Eigenschaften der biologischen Sequenz, Ableitungen der biologischen Sequenz, Annotationen zu der biologischen Sequenz sowie Ordnungs- und Distanzbeziehungen zwischen Merkmalen,

wobei mindestens ein Komponentenmerkmal ist:

(i) eine eindeutige Sequenzzeichenfolge, die in einer Mehrzahl von Bewegungen eines Sliding Window erscheint, das benachbarte Einheiten innerhalb einer bestimmten Distanz von Einheiten einer Basispositionseinheit in der Datenstruktur biologischer Sequenz umfasst; oder

(ii) eine eindeutige Sequenzzeichenfolge mit einer gegebenen ganzzahligen Länge aufeinanderfolgender Einheiten der Datenstruktur biologischer Sequenz, wobei die eindeutige Sequenzzeichenfolge optional eine eindeutige Sequenzzeichenfolge mit einer größeren gegebenen ganzzahligen Länge aufeinanderfolgender Einheiten der Datenstruktur biologischer Sequenz umfasst, die durch Zusammenführen benachbarter eindeutiger Sequenzzeichenfolgen mit einer kleineren ganzzahligen Länge aufeinanderfolgender Einheiten der Datenstruktur biologischer Sequenz erzeugt werden; oder

(iii) mindestens eines aus: mindestens einem Muster in der Datenstruktur biologischer Sequenz und mindestens einem Muster in mindestens einer Position der Datenstruktur biologischer Sequenz.

2. Computerimplementiertes Verfahren nach Anspruch 1, wobei ein Wert von mindestens einem Komponentenmerkmalseintrag der Fingerabdruckdatenstruktur mindestens eines umfasst aus: einer Zählung des Merkmals in der Datenstruktur biologischer Sequenz; einer Zeichenfolge, die den mindestens einen Komponentenmerkmalseintrag darstellt; und einem fortlaufenden Zahlenwert, der den mindestens einen Komponentenmerkmalseintrag darstellt.

3. Computerimplementiertes Verfahren nach Anspruch 1 oder 2, wobei ein Wert von mindestens einem Komponentenmerkmalseintrag der Fingerabdruckdatenstruktur einen Wert umfasst, der die biologische Sequenz als Ganzes charakterisiert.

4. Computerimplementiertes Verfahren nach einem der Ansprüche 1 bis 3, wobei mindestens ein Komponentenmerkmal der Fingerabdruckdatenstruktur ein Merkmal umfasst, das eine Annotation zu der biologischen Sequenz darstellt.

5. Computerimplementiertes Verfahren nach einem der Ansprüche 1 bis 4, wobei mindestens ein Komponentenmerkmal der Fingerabdruckdatenstruktur ein Merkmal umfasst, das mindestens eines aus einer Ordnungsbeziehung oder einer Distanzbeziehung zwischen zwei oder mehr anderen Komponentenmerkmalen der biologischen Sequenz darstellt.

6. Computerimplementiertes Verfahren zum Vergleichen einer ersten biologischen Sequenz mit einer zweiten biologischen Sequenz, wobei das Verfahren umfasst:

Bilden einer ersten Fingerabdruckdatenstruktur, die die erste biologische Sequenz darstellt, und einer zweiten Fingerabdruckdatenstruktur, die die zweite biologische Sequenz darstellt, mit dem computerimplementierten Verfahren nach einem der Ansprüche 1 bis 5; und

Vergleichen der ersten Fingerabdruckdatenstruktur und der zweiten Fingerabdruckdatenstruktur durch Ermitteln einer Ähnlichkeit oder Distanz zwischen den Merkmalskomponenten der ersten Fingerabdruckdatenstruktur und der zweiten Fingerabdruckdatenstruktur.

7. Computerimplementiertes Verfahren nach Anspruch 6, wobei das Ermitteln einer Ähnlichkeit oder Distanz auf dem Jaccard-Ähnlichkeitskoeffizienten der ersten Fingerabdruckdatenstruktur und der zweiten Fingerabdruckdatenstruktur basiert.

8. Computerimplementiertes Verfahren nach Anspruch 7, wobei das Ermitteln einer Ähnlichkeit oder Distanz das Ermitteln einer Distanz umfasst, indem der Wert jedes Bits in einem ersten Bitsatz der ersten biologischen Sequenz mit dem Wert jedes Bits in einem zweiten Bitsatz der zweiten biologischen Sequenz verglichen wird, wobei die Distanz eine Ähnlichkeit zwischen dem ersten Bitsatz und dem zweiten Bitsatz darstellt.

9. Computerimplementiertes Verfahren nach Anspruch 8, wobei die Distanz eine Tanimoto-Distanz ist.

10. Computersystem, das umfasst:

einen Prozessor; und

einen Speicher mit darauf gespeicherten Computercodeanweisungen, wobei der Prozessor und der Speicher mit den Computercodeanweisungen konfiguriert sind, um das Verfahren nach einem der Ansprüche 1 bis 5 zu implementieren.

11. Computersystem, das umfasst:

einen Prozessor; und

einen Speicher mit darauf gespeicherten Computercodeanweisungen, wobei der Prozessor und der Speicher mit den Computercodeanweisungen konfiguriert sind, um das Verfahren nach einem der Ansprüche 6 bis 9 zu implementieren.

12. Computerprogramm, das Anweisungen umfasst, um einen Prozessor dazu zu veranlassen, das Verfahren nach einem der Ansprüche 1 bis 5 auszuführen.

13. Computerprogramm, das Anweisungen umfasst, um einen Prozessor dazu zu veranlassen, das Verfahren nach einem der Ansprüche 6 bis 9 auszuführen.

14. Nichttransitorisches computerlesbares Medium, auf dem das Computerprogramm nach Anspruch 12 aufgezeichnet ist.

15. Nichttransitorisches computerlesbares Medium, auf dem das Computerprogramm nach Anspruch 13 aufgezeichnet ist.

**Revendications**

1. Procédé implémenté par ordinateur pour former une structure de données d'empreinte représentant une séquence biologique, le procédé implémenté par ordinateur comprenant :

pour chaque caractéristique de composant d'une pluralité de caractéristiques de composants à utiliser dans la structure de données d'empreinte, l'interrogation d'une structure de données de séquence biologique représentant la séquence biologique afin de déterminer une valeur de la caractéristique de composant dans la structure de données de séquence biologique, la valeur indiquant :

- une présence ou absence de la caractéristique de composant dans la séquence biologique, ou
- une valeur réelle de la caractéristique de composant ; et

l'ajout d'une entrée de caractéristique de composant à la structure de données d'empreinte qui correspond au résultat de l'interrogation de la structure de données de séquence biologique pour la caractéristique de composant, l'entrée de caractéristique de composant ayant la valeur déterminée de la caractéristique de composant ;

au moins une partie des entrées de caractéristiques de composants ajoutées à la structure de données d'empreinte étant indiquée dans des bits de caractéristiques d'un ensemble de bits, chaque bit de caractéristique correspondant à une caractéristique de composant respective ayant une valeur qui indique une présence ou absence de la caractéristique de composant respective dans la séquence biologique,

dans lequel les caractéristiques de composants comportent au moins l'un de ce qui suit : des propriétés de la séquence biologique, des dérivations de la séquence biologique, des annotations de la séquence biologique et des relations d'ordre et de distance entre les caractéristiques,

dans lequel au moins une caractéristique de composant est :

(i) une chaîne de séquence unique qui apparaît dans une pluralité de déplacements d'une fenêtre glissante comprenant des unités voisines à une distance donnée d'unités d'une unité de position de base dans la structure de données de séquence biologique ; ou

(ii) une chaîne de séquence unique d'une longueur d'entier donnée d'unités successives de la structure de données de séquence biologique, dans lequel, optionnellement, la chaîne de séquence unique comprend une chaîne de séquence unique d'une longueur d'entier donnée plus élevée d'unités successives de la structure de données de séquence biologique créée en fusionnant les chaînes de séquences uniques voisines d'une longueur d'entier moins élevée d'unités successives de la structure de données de séquence biologique ; ou

(iii) au moins l'un de ce qui suit : au moins un motif dans la structure de données de séquence biologique, et au moins un motif à au moins une position de la structure de données de séquence biologique.

2. Procédé implémenté par ordinateur selon la revendication 1, dans lequel une valeur d'au moins une entrée de caractéristique de composant de la structure de données d'empreinte comprend au moins l'un de ce qui suit : un décompte de la caractéristique dans la structure de données de séquence biologique ; une chaîne qui représente l'au moins une entrée de caractéristique de composant ; et une valeur à nombre continu qui représente l'au moins une entrée de caractéristique de composant.

3. Procédé implémenté par ordinateur selon la revendication 1 ou 2, dans lequel une valeur d'au moins une entrée de caractéristique de composant de la structure de données d'empreinte comprend une valeur qui caractérise la séquence biologique dans son ensemble.

4. Procédé implémenté par ordinateur selon l'une quelconque des revendications 1 à 3, dans lequel au moins une caractéristique de composant de la structure de données d'empreinte comprend une caractéristique qui représente une annotation de la séquence biologique.

5. Procédé implémenté par ordinateur selon l'une quelconque des revendications 1 à 4, dans lequel au moins une caractéristique de composant de la structure de données d'empreinte comprend une caractéristique qui représente au moins l'une d'une relation d'ordre ou d'une relation de distance entre deux ou plus autres caractéristiques de composants de la séquence biologique.

6. Procédé implémenté par ordinateur destiné à comparer une première séquence biologique avec une deuxième séquence biologique, le procédé comprenant :

la formation d'une première structure de données d'empreinte qui représente la première séquence biologique, et d'une deuxième structure de données d'empreinte qui représente la deuxième séquence biologique avec le procédé informatique selon l'une quelconque des revendications 1 à 5 ; et

la comparaison de la première structure de données d'empreinte et de la deuxième structure de données d'empreinte en déterminant la similarité ou la distance entre les caractéristiques de composants de la première structure de données d'empreinte et de la deuxième structure de données d'empreinte.

7. Procédé implémenté par ordinateur selon la revendication 6, dans lequel la détermination de similarité ou de distance repose sur le coefficient de similarité de Jaccard de la première structure de données d'empreinte et de la deuxième structure de données d'empreinte.

8. Procédé implémenté par ordinateur selon la revendication 7, dans lequel la détermination de la similarité ou de la distance comprend la détermination d'une distance en comparant la valeur de chaque bit dans un premier ensemble

**EP 3 477 648 B1**

de bits de la première séquence biologique avec la valeur de chaque bit dans un deuxième ensemble de bits de la deuxième séquence biologique, la distance représentant la similarité entre le premier ensemble de bits et le deuxième ensemble de bits.

9. Procédé implémenté par ordinateur selon la revendication 8, dans lequel la distance est une distance de Tanimoto.

10. Système informatique comprenant :

un processeur ; et
une mémoire avec des instructions de code informatique stockées dessus, le processeur et la mémoire, avec les instructions de code informatique, étant configurés pour mettre en œuvre le procédé selon l'une quelconque des revendications 1 à 5.

11. Système informatique comprenant :

un processeur ; et
une mémoire avec des instructions de code informatique stockées dessus, le processeur et la mémoire, avec les instructions de code informatique, étant configurés pour mettre en œuvre le procédé selon l'une quelconque des revendications 6 à 9.

12. Programme d'ordinateur comprenant des instructions destinées à permettre à un processeur d'exécuter le procédé selon l'une quelconque des revendications 1 à 5.

13. Programme d'ordinateur comprenant des instructions destinées à permettre à un processeur d'exécuter le procédé selon l'une quelconque des revendications 6 à 9.

14. Support non transitoire lisible par un ordinateur stockant le programme informatique selon la revendication 12.

15. Support non transitoire lisible par un ordinateur stockant le programme informatique selon la revendication 13.

Biological Sequence Bitset Fingerprint Data
Structure System
100

Processor
102

Memory
104

Component Features
in Fingerprint Data
Structure
110

Bitset 118

Feature Bit 1

Feature Bit 2

•

•

•

Feature Bit N

Sequence Evaluation Module
106

Component Feature Editor
Module
108

Query regarding
Presence or Value
of Component
Feature
116

Biological Sequence
Data Structure
112

Sequence Data 1

Sequence Data 2

•

•

•

Sequence Data N

Add Feature Entry
for Result of Query
114

FIG. 1

Sequence Evaluation Module
206

Primary Feature
Module
220

Secondary Feature
Module
224

Feature calculated or
derived from
Sequence  226

Tertiary Feature
Module
228

Order or
Distance 234

Quaternary Feature
Module
232

Biological Sequence
Data Structure
212

Value Characterizing
Sequence as a Whole
222

Sequence Data 1

Sequence Data 2

●
●  229
●

Sequence Data N

Annotation of Biological
Sequence
230

Other Characteristics of
Biological Sequence
264

FIG. 2

| Secondary Feature Module 324 | | Biological Sequence Data Structure 312 |
|---|---|---|
| Sliding Window Module 336 | ⟷ | Sequence Data 1<br>Sequence Data 2 |
| Unique Sequence Module 338 | | • |
| Extended Connectivity Module 340 | ⟷ | • 329<br>•<br>•<br>•<br>•<br>• |
| Pattern Position Module 342 | ⟷ | •<br>•<br>• |
| Pattern Presence Module 344 | ⟷ | •<br>Sequence Data N |

FIG. 3

Query Biological Sequence Data Structure regarding Presence or Value
of Component Feature in Biological Sequence Data Structure

<u>405</u>

Add Component Feature Entry to Fingerprint Data Structure
corresponding to result of Querying Biological Sequence Data Structure
for Component Feature;
Component Feature Entries of Fingerprint Data Structure comprise
Feature Bits of a Bitset

<u>407</u>

FIG. 4

FIG. 5

Start

Biological
Sequence

609

Create Fingerprint,
Initializing all bits to
0

621

617

More Features
to Determine?

No

Output Final
Fingerprint

619

End

No

Calculate Next
Feature

613

623

Feature Found in
Sequence?

Yes

Set Feature Bit to 1

615

FIG. 6

FIG. 7

**1-mer**
ATGCATAAT
Unique Features:
- A
- T
- G
- C

**2-mer**
ATGCATAAT
Unique Features:
- AT
- TG
- GC
- CA
- TA
- AA

**3-mer**
ATGCATAAT
Unique Features:
- ATG
- TGC
- GCA
- CAT
- TAA
- AAT

**4-mer**
ATGCATAAT
Unique Features:
- ATGC
- TGCA
- GCAT
- CATA
- ATAA
- TAAT

**5-mer**
ATGCATAAT
Unique Features:
- ATGCA
- TGCAT
- GCATA
- CATAA
- ATAAT

FIG. 8

| **Step 1** | **Step 2** | **Step 3** | **Step 4** |
|---|---|---|---|
| A̲T̲G̲C̲A̲T̲A̲A̲T | A̲T̲G̲C̲A̲T̲A̲A̲T | A̲T̲G̲C̲A̲T̲A̲A̲T | A̲T̲G̲C̲A̲T̲A̲A̲T |
| Unique Features: | Unique Features: | Unique Features: | Unique Features: |
| • A | • AT | • ATGC | • ATGCATAA |
| • T | • GC | • ATAA | |
| • G | • AA | | |
| • C | | | |

FIG. 9

Component Features
in Fingerprint Data
Structure
1010

Bitset 1018

Feature Bit 1

Feature Bit 2

•
•
•
•
•
•
•
•
•

Feature Bit N

Similarity Evaluation Module
1046

Sequence Masking
1056

Analysis Module
1048

Assay Correlation
1058

Machine Learning Module
1050

SAR/QSAR
1060

Searching Module
1052

AND, OR, FOLLOWING,
BUT NOT, Other Terms 1062

Metagenomics Module
1054

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0106454 A1

- US 20170270240 A1

**Non-patent literature cited in the description**

- Pattern Matching. **MARKEL S.** ; **RAJAPAKSE V.** In Silico Technology in Drug Target Identification and Validation. Marcel Dekker, 2006
- **DAVID ROGERS** ; **MATHEW HAHN**. Extended-Connectivity Fingerprints. *Journal of Chemical Information and Modeling*, 2010, vol. 50 (5), 742-754

- **DAVID J. ROGERS** ; **TAFFEE T. TANIMOTO**. A Computer Program for Classifying Plants. *Science*, 1960, vol. 132 (3434), 1115-1118